# EUROPEAN PATENT APPLICATION

(11) **EP 3 513 726 A1**
(43) Date of publication of application: **24.07.2019**
(21) Application number: 17850877.6
(22) Date of filing: 12.09.2017
(51) Int. Cl.: A61B 5/1455, A61B 5/00, A61B 5/0245

(54) **BIOLOGICAL INFORMATION MEASUREMENT DEVICE**

(30) Priority: 14.09.2016 JP 2016179380
(71) Applicant: Dynamic Brain Lab, LLC., Tokyo 162-0842 (JP)
(72) Inventor: VALENZI Stefano, Tokyo 134-0044 (JP); JURICA Peter, Wako-shi Saitama 351-0104 (JP)
(74) Representative: Cabinet Nuss
(86) International application number: PCT/JP2017/032853
(87) International publication number: WO 2018/051975

(57) **Abstract**

Provided is a biometric information measuring device 100, including a sensor module 20 that has N light-emitting elements 21 (N being an integer of 3 or more) that emit light having different wavelengths, and N-1 light-receiving elements 22 that receive light emitted from the light-emitting elements 21 and reflected by a biological tissue 10 and that output signals corresponding to the light having different wavelengths; and a signal processing unit that executes signal processing based on the output of the light-receiving elements 22, in which at least two light-emitting elements 21 are disposed on a circumference at a constant radius from light-receiving elements 22 of the sensor module 20 as a center.

## Description

### [Technical Field]

The present invention relates to a biometric information measuring device, and particularly relates to an optical biometric information measuring device for measuring pieces of biometric information.

### [Background Art]

In the related art, pulsimeters, pulse oximeters, and the like are known as optical biometric information measuring devices. Pulse oximeters non-invasively measure a blood oxygen saturation level of a living body. The principle is that, using a difference in absorbance with respect to light having a predetermined wavelength of oxidized hemoglobin and reduced hemoglobin, a light-emitting element is made to emit light having different wavelengths to a living body, and the absorbance with respect to the light is detected to measure a blood oxygen saturation level (refer to, for example, Patent Literature 1).

### [Citation List]

### [Patent Literature]

### [Patent Literature 1]

Japanese Unexamined Patent Application, First Publication No. 3116-000255

### [Summary of Invention]

### [Technical Problem]

Meanwhile, although the pulse oximeter as shown in Patent Literature 1 includes two light-emitting elements and one light-receiving element, it is difficult to acquire biometric information including a plurality of blood components, which are for measuring a blood oxygen saturation level and a blood glucose level, for example. In order to acquire pieces of biometric information, each of separate light-emitting elements and light-receiving elements are required.

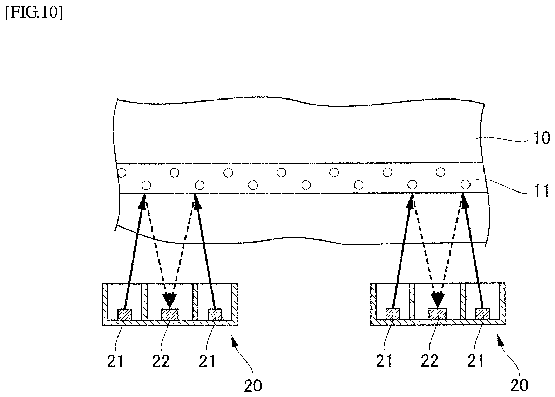

Accordingly, a pulse oximeter having two sensor modules as shown in Fig. 10 may be conceived, but there is a problem of this pulse oximeter needing to include four light-emitting elements, which makes it hard to realize reduction in size of a device and power saving.

An object of the present invention is to provide a biometric information measuring device in which pieces of biometric information are able to be acquired, while realizing reduction in size thereof and power saving with a simple configuration.

### [Solution to Problem]

In order to achieve the above object, a biometric information measuring device according to one aspect of the present invention includes:
a sensor module that has N light-emitting elements (N being an integer of 3 or more) that emit light having different wavelengths, and N-1 light-receiving elements that receive light emitted from the light-emitting elements and reflected by a living body and that output signals corresponding to the light having different wavelengths; and
a signal processing unit that executes signal processing based on the output of the light-receiving elements,
in which at least two light-emitting elements are disposed on a circumference at a constant radius from light-receiving elements of the sensor module as a center.

In order to achieve the above object, a biometric information measuring device according to another aspect of the present invention includes:
a sensor module that includes
   a first light-emitting element that emits light having a first wavelength,
   a second light-emitting element that emits light having a second wavelength,
   a third light-emitting element that emits light having a third wavelength,
   a first light-receiving element that receives light emitted from the first light-emitting element and the second light-emitting element and reflected by a living body, and that outputs signals corresponding to the light having different wavelengths, and
   a second light-receiving element that receives light emitted from the second light-emitting element and the third light-emitting element and reflected by a living body, and that outputs signals corresponding to the light having different wavelengths; and
a signal processing unit that executes signal processing based on the output of the light-receiving elements,
in which the first light-emitting element and the second light-emitting element are disposed on a circumference at a constant radius from the first light-receiving element of the sensor module as a center, and the second light-emitting element and the third light-emitting element are disposed on a circumference at a constant radius from the second light-receiving element as a center.

### [Advantageous Effects of Invention]

According to the present invention, it is possible to provide a biometric information measuring device in which pieces of biometric information are able to be acquired, while realizing reduction in size thereof and power saving with a simple configuration.

### [Brief Description of Drawings]

Fig. 1 is a view showing a configuration example of a sensor module in a biometric information measuring device according to a first embodiment of the present invention.
Fig. 2A is a schematic plan view showing the disposition of light-emitting elements and light-receiving elements of the sensor module in the biometric information measuring device according to the first embodiment of the present invention.
Fig. 2B is a schematic plan view showing the disposition of the light-emitting elements and the light-receiving elements of the sensor module in the biometric information measuring device according to the first embodiment of the present invention.
Fig. 3 is a block diagram showing a functional configuration of a biometric information measuring device according to a second embodiment of the present invention.
Fig. 4 is a block diagram showing a functional configuration of a biometric information measuring device according to a third embodiment of the present invention.
Fig. 5 is a view showing an external configuration as an example of the biometric information measuring device according to the third embodiment of the present invention.
Fig. 6 is a view showing an external configuration as an example of a biometric information measuring device according to a fourth embodiment of the present invention.
Fig. 7 is a view showing an external configuration as an example of a biometric information measuring device according to a fifth embodiment of the present invention.
Fig. 8 is a schematic view in which the biometric information detection device according to the fourth embodiment of the present invention is attached to an external device.
Fig. 9 is a schematic view showing a band part for allowing the biometric information measuring device of the present invention to be worn on and fixed to a biological tissue.
Fig. 10 is a diagram showing a configuration example of a sensor module in a biometric information measuring device of the related art.

### [Description of Embodiments]

Hereinafter, various embodiments of a biometric information measuring device according to the present invention will be described in detail below with reference to the drawings.

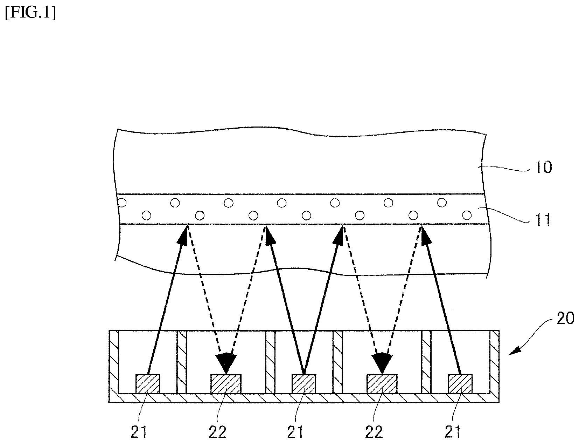

Fig. 1 is a view showing a configuration example of a sensor module in a biometric information measuring device according to a first embodiment of the present invention. The sensor module 20 of the present invention includes three light-emitting elements 21 and two light-receiving elements 22. The sensor module 20 of the present invention is characterized in having one less light-emitting element 21 than that shown in Fig. 10 described above.

In a biometric information measuring device 100 including the sensor module 20 shown in Fig. 1, the three light-emitting elements 21 emit light having different wavelengths to a biological tissue 10, and two common light-receiving elements 22 receive the reflected light and output a signal corresponding to the light having the wavelengths. Accordingly, while realizing absorption of signal fluctuation in accordance with the body movement of a subject to be measured, and acquisition of a blood oxygen saturation level, which are characteristics of a pulse oximeter, it is possible to acquire biometric information including a plurality of blood components so that a blood glucose level is also measured. In addition, it is possible to measure the same components of a living body and calculate an average value thereof, and furthermore, when abnormalities occur in one of light-receiving elements but the other light-receiving element is normal, it is possible to acquire desired biometric information. Therefore, improvement in reliability of measurement values is realized. Furthermore, compared with devices of the related art, using a simple configuration with one less light emitting element 21, the space necessary for mounting the light emitting element 21, the voltage loss in the driving circuit, and the like in the related art are eliminated, and reduction in size and power saving in the device are able to be realized.

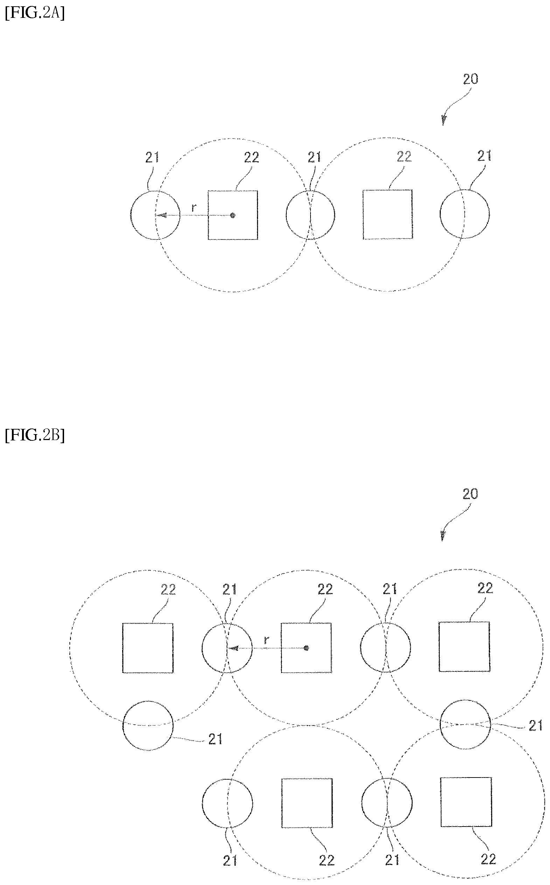

Figs. 2A and 2B are schematic plan views showing the disposition of the light-emitting elements and the light-receiving elements of the sensor module in the biometric information measuring device of the present invention. The sensor module 20 includes N light-emitting elements 21 and N-1 light-receiving elements 22. Fig. 2A shows an example of disposition when N is 3, and Fig. 2B shows an example of disposition when N is 5.

The first light-emitting element 21 and the second light-emitting element 21 are disposed on a circumference at a radius r indicated by a dashed line from the first light-receiving element 22 as a center. The second light-emitting element 21 and the third light-emitting element 21 are disposed on a circumference at a radius r indicated by a dashed line from the second light-receiving element 22 as a center. The radius r indicates center spacings between the two light-emitting elements 21 and the common light-receiving element 22. In order to realize reduction in size of the sensor module 20, the radius r is preferably within 1 mm. As shown in Fig. 2A, the first light-emitting element, the second light-emitting element, the third light-emitting element, the first light-receiving element, and the second light-receiving element may be disposed on the same straight line.

In addition, in Fig. 2B, the light-emitting element 21 and the light-receiving element 22 are disposed such that the disposition form is in two rows, but the disposition is not necessarily in two rows. For example, the disposition form may be in one row or three rows. In short, the light-emitting elements 21 may be provided around the light-receiving element so that a distance between the light-emitting elements 21 and the common light-receiving element 22 is the same.

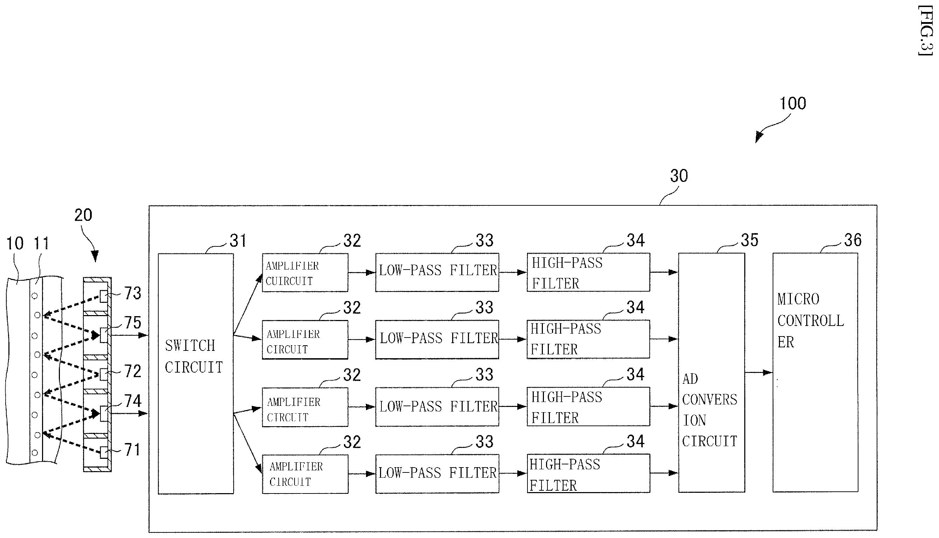

Fig. 3 is a block diagram showing a functional configuration of a biometric information measuring device according to a second embodiment of the present invention. The biometric information measuring device 100 according to the present embodiment includes a sensor module 20 and a signal processing unit 30. A light-emitting element 21 includes a red LED 71 that emits a wavelength in a red region, an infrared LED 72 that emits a wavelength in an infrared region, and a near-infrared LED 73 that emits a wavelength in a near-infrared region. A light-receiving element includes a photodiode 74 that receives light having a wavelength between the red region and the infrared region, and a photodiode 75 that receives light having a wavelength between the infrared region and the near-infrared region. The signal processing unit 30 includes a switch circuit 31, an amplifier circuit 32, a low-pass filter 33, a high-pass filter 34, an AD conversion circuit 35, and a microcontroller 36. A wavelength region received by the photodiode 74 is preferably 600 to 1000 nm. A wavelength region received by the photodiode 75 is preferably 900 to 1600 nm.

The red LED 71, the infrared LED 72, and the near-infrared LED 73 of the sensor module 20 selectively perform emission according to the switch circuit 31, and irradiate a biological tissue 10 with light having different wavelengths. The photodiodes 74 and 75 of the sensor module 20 receive the light reflected by the biological tissue 10, and output a signal corresponding to the wavelengths. The switch circuit 31 distributes and outputs the signals output from the photodiodes 74 and 75 to the amplifier circuits 32, respectively. Each of the amplifier circuits 32 includes a hold circuit for holding a voltage at the time of opening in order to prevent an output voltage of the amplifier circuit 32 from being lowered by the switch circuit 31 at the time of opening. The low-pass filter 33 performs low-pass filtering of signals with a predetermined cutoff frequency, which are output from the amplifier circuit 32, and outputting of the signal. The high-pass filter 34 performs high-pass filtering of signals with a predetermined cutoff frequency, and outputting of the signal. The AD conversion circuit 35 converts a signal output from the high-pass filter 34 from an analog signal to a digital signal and outputs the signal. The microcontroller 36 executes signal processing such as waveform shaping on the basis of a digital signal output from the AD conversion circuit 35, and outputs biometric information as a measurement result. The acquired biometric information includes one or more of a pulse, a blood oxygen level, or a blood glucose level.

The various processes described above need not be performed only in the time series in accordance with this description, and also may be performed in parallel or separately as necessary or depending on the processing capability of the device performing the process.

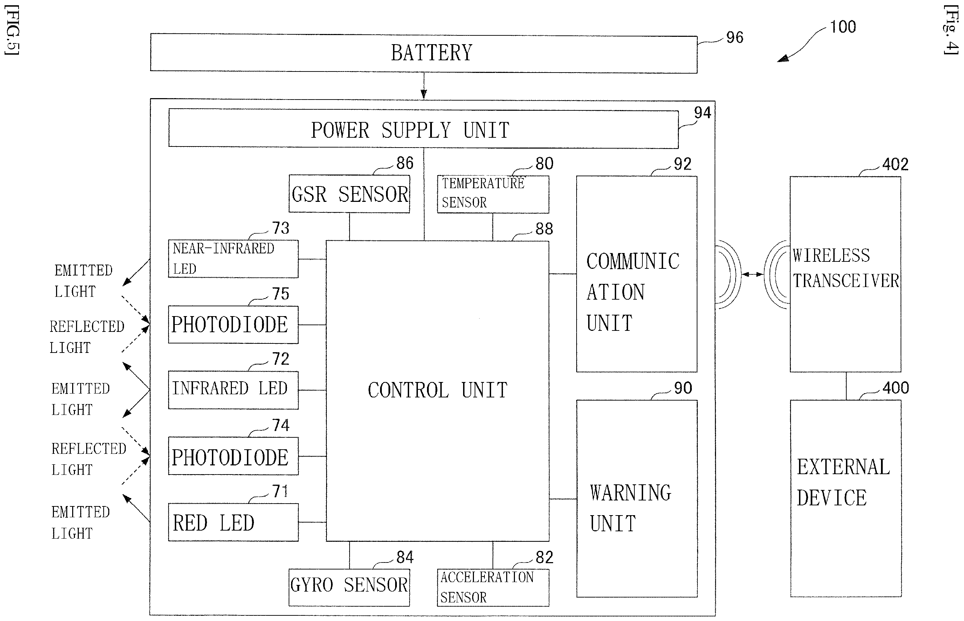

Fig. 4 is a block diagram showing a functional configuration of a biometric information measuring device according to a third embodiment of the present invention. In addition to the constituent elements of the biometric information measuring device 100 of the second embodiment, a biometric information measuring device 100 according to the third embodiment further includes a temperature sensor 80, an acceleration sensor 82, a gyro sensor 84, a GSR sensor 86, a control unit 88, a warning unit 90, a communication unit 92, and a power supply unit 94.

The temperature sensor 80 has a function of detecting a living body temperature of a subject to be measured and an external temperature. The temperature sensor 80 is connected to the control unit 88 and outputs detected signals to the control unit 88.

The acceleration sensor 82 and the gyro sensor 84 have a function of detecting the body motion of a subject to be measured. The acceleration sensor 82 and the gyro sensor 84 are connected to the control unit 88 and output detected signals to the control unit 88. As in the present embodiment, the biometric information measuring device 100 is configured to include both the acceleration sensor 82 and the gyro sensor 84. Detection results from both sensors may be used in combination, or results from one of the sensors may be used.

The GSR sensor 86 has a function of detecting a value of galvanic skin response (GSR) of a subject to be measured. The GSR sensor 86 is connected to the control unit 88 and outputs detected signals to the control unit 88.

The control unit 88 is connected to the above-described various sensors. The control unit 88 includes a microcontroller 36 and is a control device and a signal processing device that control the overall operation of the biometric information measuring device 100. The control unit 88 also has a function of storing a control program to be executed, processing results, various data, and the like. The control unit 88 controls various sensors to perform operations of acquiring, analyzing, and outputting of measurement data of biometric information. The measurement data may be transmitted to an external device 400 via the communication unit 92 each time a signal detected by various sensors is acquired, or may be temporarily stored in the control unit 88 to be periodically transmitted. In addition, in a case of transmitting the acquired measurement data to the external device 400, data analysis of the above-described measurement data may be performed by the external device 400.

Because signals output from the various sensors to the control unit 88 are analog signals, the control unit 88 has an AD conversion function for converting input analog signals into digital signals. However, in a case where the input analog signals are directly transmitted to the external device 400, the control unit 88 is not required to have the AD conversion function.

The obtained biometric information includes, for example, perspiration, a heartbeat, a pulse wave, skin temperature, galvanic skin response, skin resistance value, blood oxygen saturation level, blood glucose level, and the like, and may be appropriately adopted according to the purpose of using the biometric information measuring device 100.

The warning unit 90 includes a light output section that outputs predetermined light and a sound output section that outputs predetermined sound. The warning unit 90 has a function of emitting predetermined light or sound to a subject to be measured or the like on the basis of the detection that the acquired biometric information is equal to or less than a predetermined threshold value. In addition, in a case where measurement has not started for a certain period of time after the measurement of biometric information is stopped by a subject to be measured, light or sound may be emitted so that the warning unit 90 prompts restarting of the measurement.

The communication unit 92 has a function of transmitting measurement data signal-processed by the control unit 88 to the external device 400 wirelessly or via a wire. In addition, the communication unit 92 has a function of transmitting a signal notifying the external device 400 of abnormalities on the basis of the detection that the acquired biometric information is equal to or less than a predetermined threshold value. In the present embodiment, the signal is transmitted to the external device 400 including a wireless transceiver 402 by a method such as infrared communication or Bluetooth (registered trademark).

In addition, the communication unit 92 may transmit the signal to an external information device via a cable 98. In this case, power is supplied using a power supply line of the cable 98, and therefore power is supplied at the same time as when the communication unit 92 is connected to the external device 400 via the cable 98. The cable 98 may be a USB cable.

The power supply unit 94 includes a connection terminal for connection to an external battery 96 which is detachable or the external device 400 capable of supplying power, a power supply circuit, and a charging circuit. This connection terminal may be a USB terminal.

The external device 400 includes a wireless transceiver unit 402. Examples thereof include a mobile terminal, a tablet terminal, and the like. The external device 400 preferably includes a display unit capable of displaying measurement data and the like received from the communication unit 92. Accordingly, a subject on which measurement is to be performed using the biometric information measuring device 100 can check his or her current or previous biometric information according to numerical values.

The biometric information measuring device 100 and the external device 400 are not limited to the configuration of the present embodiment, and various modifications such as omitting some of these constituent elements or adding other constituent elements can be made.

Fig. 5 is a view showing an external configuration as an example of the biometric information measuring device according to the third embodiment of the present invention. The external appearance of the biometric information measuring device 100 according to the present embodiment shows a housing 97, two photodiodes 74 and 75, a GSR sensor 86, warning units 90 for outputting predetermined light and sound, and an external battery 96 which is detachable. In the present embodiment, the housing 97 is a substantially rectangular parallelepiped box-like member and has a light-transmission window. The light-transmission window is formed of a member that transmits light, and the light transmitted through the light-transmission window is received by the light-receiving element of the sensor module. The interior of the housing 97 is a disposition space for various sensors and the like. The biometric information measuring device 100 of the present embodiment is a wearable-type device having no wired cable. A thickness of the housing 97 is preferably thin, and is preferably 3 mm or less.

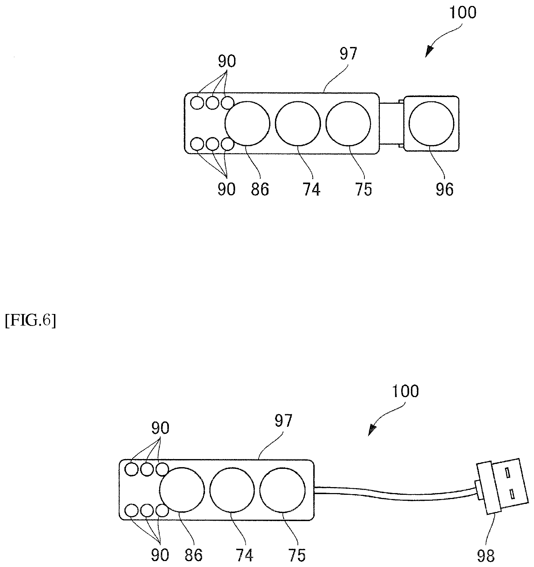

Fig. 6 is a view showing an external configuration as an example of the biometric information measuring device according to a fourth embodiment of the present invention. The external appearance of a biometric information measuring device 100 according to the present embodiment shows a housing 97, two photodiodes 74 and 75, a GSR sensor 86, warning units 90 for outputting predetermined light and sound, and a cable 98. An external device 400 supplies power to the biometric information measuring device 100 according to the present embodiment via the cable 98. In addition, data can be transmitted to the external device 400 via the cable 98. The cable 98 may be a USB cable. The biometric information measuring device 100 of the present embodiment is a portable-type device.

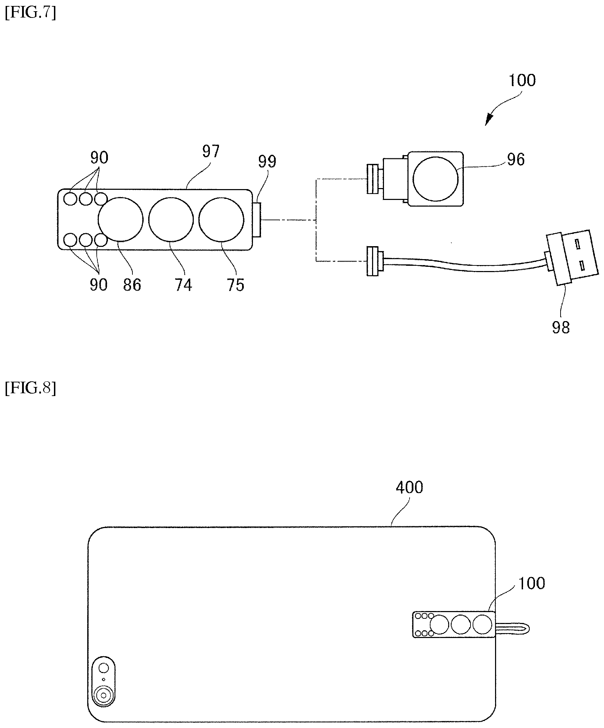

Fig. 7 is a view showing an external configuration as an example of the biometric information measuring device according to the fourth embodiment of the present invention. The external appearance of the biometric information measuring device 100 according to the present embodiment shows a housing 97, two photodiodes 74 and 75, a GSR sensor 86, warning units 90 for outputting predetermined light and sound, and a connection terminal 99. The connection terminal 99 can be connected to any of a battery 96 and the external device 400. The connection terminal 99 may be a USB connection terminal.

The biometric information measuring device 100 of the present embodiment may be any of the wearable-type shown in the third embodiment, or the portable-type shown in the fourth embodiment. According to the present embodiment, there is no need to separately manufacture each of a wearable-type device and a portable-type device. Therefore, it is possible to reduce man-hours and manufacturing costs.

Fig. 8 is a schematic view in which the biometric information detection device according to the fourth embodiment of the present invention is attached to an external device. When the biometric information measuring device 100 is connected to the external device 400 via the cable 98, power is supplied from the external device 400, or data communication is performed between the biometric information measuring device 100 and the external device 400. The present embodiment is configured such that, in a case where the external device 400 includes a display unit, measurement data obtained by the biometric information measuring device 100 is displayed on the external device 400. Reduction in size has been realized with respect to the sensor module 20 of the present invention, and thus a thickness thereof is thin. Accordingly, the housing 97 of the biometric information measuring device 100 according to the present embodiment is also relatively reduced in size and becomes thin. For this reason, as shown in Fig. 8, the biometric information measuring device 100 according to the present embodiment can be provided on an upper surface of the external device 400, and does not restrict movement of a subject to be measured, and therefore is excellent in portability.

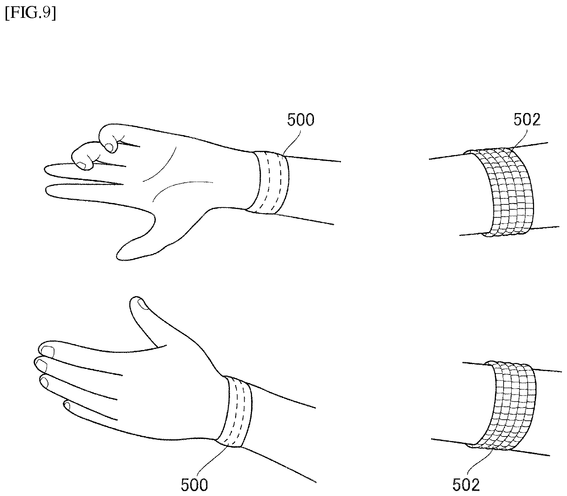

Fig. 9 is a schematic view showing a band part for allowing the biometric information measuring device of the present invention to be worn on and fixed to a biological tissue. The band part includes a first band part 500 and a second band part 502. The first band part 500 detachably holds the biometric information measuring device 100, and has an inner surface so that the biometric information measuring device 100 is brought into contact with a biological tissue 10. In the present drawing, the first band part 500 includes a hole portion capable of storing the biometric information measuring device 100, for example, in a dotted line portion (shown in the drawing) of the first band part 500. The second band part 502 is worn on the first band part and has a stretchable light-shielding member that shields the surface brought into contact with the biological tissue 10 or directly shields leakage of light.

In the present drawing, the band-type biometric information measuring device 100 wound around the wrist of a living body to be worn has been described as an example, but the biometric information measuring device 100 to which the present invention can be applied is not limited thereto. For example, the present invention can be applied to a finger-wearing type biometric information measuring device 100 in which biometric information is measured by wearing the device on a fingertip, or a spectacle-type biometric information measuring device 100 in which the measuring device is stored in a frame of spectacles.

The above-described embodiments are merely examples specified for carrying out the present invention, and the technical scope of the present invention should not be interpreted limitedly by these embodiments. That is, the present invention can be carried out in various forms without departing from the gist or the main features thereof.

In addition, it is obvious that the configurations of the embodiments and operations described above can be arbitrarily combined to carry out the present invention as long as they do not contradict each other.

The present application claims priority right based on Japanese Patent Application No. 2016-179380 filed September 14, 2016, and incorporates all contents of description described in the Japanese Patent Application.

### [Reference Signs List]

10 Biological tissue
11 Blood
20 Sensor module
21 Light-emitting element
22 Light-receiving element
30 Signal processing unit
71 Red LED
72 Infrared LED
73 Near-infrared LED
74 Photodiode
75 Photodiode
80 Temperature sensor
82 Acceleration sensor
84 Gyro sensor
86 GSR sensor
88 Control unit
90 Warning unit
92 Communication unit
94 Power supply unit
96 Battery
98 Cable
99 Connection terminal
100 Biometric information measuring device
400 External device
402 Wireless transceiver unit
500 First band part
502 Second band part

## Claims

1. A biometric information measuring device, comprising:
a sensor module that includes
N light-emitting elements (N being an integer of 3 or more) that emit light having different wavelengths, and
N-1 light-receiving elements that receive light emitted from the light-emitting elements and reflected by a living body, and that output signals corresponding to the light having different wavelengths; and
a signal processing unit that executes signal processing based on the output of the light-receiving elements,
wherein at least two light-emitting elements are disposed on a circumference at a constant radius from light-receiving elements of the sensor module as a center.

2. The biometric information measuring device according to claim 1, wherein the light-emitting element of the sensor module has a red LED, an infrared LED, or a near-infrared LED.

3. The biometric information measuring device according to claim 1 or 2, wherein the light-receiving element of the sensor module has a photodiode that receives light having a wavelength between a red region and an infrared region, or between the infrared region and a near-infrared region.

4. A biometric information measuring device, comprising:
a sensor module that includes
a first light-emitting element that emits light having a first wavelength,
a second light-emitting element that emits light having a second wavelength,
a third light-emitting element that emits light having a third wavelength,
a first light-receiving element that receives light emitted from the first light-emitting element and the second light-emitting element and reflected by a living body, and that outputs signals corresponding to the light having different wavelengths, and
a second light-receiving element that receives light emitted from the second light-emitting element and the third light-emitting element and reflected by a living body, and that outputs signals corresponding to the light having different wavelengths; and
a signal processing unit that executes signal processing based on the output of the light-receiving elements,
wherein the first light-emitting element and the second light-emitting element are disposed on a circumference at a constant radius from the first light-receiving element of the sensor module as a center, and
the second light-emitting element and the third light-emitting element are disposed on a circumference at a constant radius from the second light-receiving element as a center.

5. The biometric information measuring device according to claim 4, wherein the first light-emitting element, the second light-emitting element, the third light-emitting element, the first light-receiving element, and the second light-receiving element are disposed on the same straight line.

6. The biometric information measuring device according to claim 4 or 5,
wherein the second wavelength is a wavelength in an infrared region, and
the second light-emitting element is an infrared LED.

7. The biometric information measuring device according to claim 4 or 5,
wherein the first wavelength is a wavelength in a red region, the second wavelength is a wavelength in the infrared region, and the third wavelength is a wavelength in a near-infrared region,
the first light-emitting element is a red LED, the second light-emitting element is an infrared LED, and the third light-emitting element is a near-infrared LED, and
the first light-receiving element is a photodiode that receives light having a wavelength between a red region and an infrared region, and the second light-receiving element is a photodiode that receives light having a wavelength between the infrared region and a near-infrared region.

8. The biometric information measuring device according to claim 7,
wherein a wavelength region received by the first light-receiving element is 600 to 1000 nm, and
a wavelength region received by the second light-receiving element is 900 to 1600 nm.

9. The biometric information measuring device according to any one of claims 1 to 8, wherein the biometric information includes at least one of a pulse, a blood oxygen level, or a blood glucose level.

10. The biometric information measuring device according to any one of claims 1 to 9, which is for acquiring biometric information, the measuring device further comprising:
a sensor unit that includes at least one of a temperature sensor for detecting a temperature of a biological tissue or an external environment, an acceleration sensor for detecting motion of a person to be measured, a gyro sensor, or a GSR sensor for detecting a galvanic skin response (GSR);
a control unit that is connected to the sensor unit;
a warning unit that includes at least one of a light output section for outputting predetermined light or a sound output section for outputting predetermined sound;
a communication unit that transmits and receives signals between the control unit and an external device; and
a power supply unit that supplies power to the control unit.

11. The biometric information measuring device according to claim 10, wherein the warning unit emits the predetermined light or sound on the basis of detection that the biometric information is equal to or less than a predetermined threshold value.

12. The biometric information measuring device according to claim 10 or 11, wherein the communication unit transmits a signal notifying the external device of abnormalities on the basis of the detection that the biometric information is equal to or less than the predetermined threshold value.

13. The biometric information measuring device according to any one of claims 10 to 12, wherein the signal in the communication unit is a wireless signal.

14. The biometric information measuring device according to any one of claims 10 to 13,
wherein the external device includes a display unit capable of displaying the biometric information, and
the display unit of the external device displays the biometric information via the communication unit of the biometric information measuring device.

15. The biometric information measuring device according to any one of claims 10 to 14,
wherein the power supply unit includes a connection terminal for power supply, and
the connection terminal is a terminal capable of being connected to any one of a battery and the external device capable of supplying power to the biometric information measuring device.

16. The biometric information measuring device according to claim 15, wherein the connection terminal of the power supply unit is a USB terminal.

17. A biometric information measuring device, comprising:
a main body unit; and
a band part that allows the main body unit to be worn and fixed to a measurement site of a living body,
wherein the main body unit includes
a sensor module that has N light-emitting elements (N being an integer of 3 or more) that emit light having different wavelengths, and N-1 light-receiving elements that receive light emitted from the light-emitting elements and reflected by a living body and that output signals corresponding to the light having different wavelengths,
a sensor unit that has at least one of a temperature sensor for detecting a temperature of a biological tissue or an external environment, an acceleration sensor for detecting motion of a person to be measured, a gyro sensor, or a GSR sensor for detecting a galvanic skin response (GSR),
a control unit that is connected to the sensor unit,
a warning unit that includes at least one of a light output section for outputting predetermined light or a sound output section for outputting predetermined sound,
a communication unit that transmits and receives signals between the control unit and an external device, and
a power supply unit that supplies power to the control unit, and
wherein the band part includes
a first band part that detachably holds the main body unit and has an inner surface so that the main body unit is brought into contact with the living body, and
a second band part that is worn on the first band part and has a stretchable light-shielding member that shields the surface brought into contact with the living body or directly shields leakage of light.
